(19) Europäisches Patentamt / European Patent Office / Office européen des brevets

(11) **EP 2 174 600 A1**

(12) **EUROPEAN PATENT APPLICATION**

(43) Date of publication:
**14.04.2010 Bulletin 2010/15**

(51) Int Cl.:
*A61B 17/22* (2006.01)   *G06T 5/00* (2006.01)
*A61B 19/00* (2006.01)

(21) Application number: **08017738.9**

(22) Date of filing: **09.10.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicant: **Dornier MedTech Systems GmbH 82234 Wessling (DE)**

(72) Inventor: **Bohris, Christian, Dr. 82152 Krailling (DE)**

(74) Representative: **Grünecker, Kinkeldey, Stockmair & Schwanhäusser Anwaltssozietät Leopoldstrasse 4 80802 München (DE)**

(54) **Method and apparatus for assigning a focus marking to a position on an ultrasound image**

(57) By means of the invention, a method as well as an apparatus for assigning a marking (26) indicating a focus of a therapeutic acoustic wave source (28) to a position on a display image (22) of an ultrasound unit (2) containing an ultrasound image (17), wherein in operation, the position of the focus relative to the ultrasound transducer (14) is determined, is to be developed which processes data generated by the ultrasound unit (2) and the pressure pulse source (28) independently from the software and data protocol of the ultrasound unit (2). This object is achieved with an apparatus and a method in which the relative position of the focus and the display image (22) are transmitted to a correlation unit (4) being separate from the ultrasound unit (2), which by means of the information contained in the display image (22) correlates the relative position of the focus to the ultrasound image (17) and assigns a focus marking (26) to a correlated position on the ultrasound image (17).

Fig. 2

## Description

The present invention relates to a method and an apparatus for assigning a marking indicating a focus of a focused therapeutic acoustic wave source to a position on a display image of an ultrasound unit containing an ultrasound image with the features of the preambles of claim 1 and claim 12. Particularly, the invention relates to a therapy device, components of which are a source for sending out therapeutic acoustic waves and an image forming modality for locating the target.

In today's medicine, as well as pulsed and continues focused acoustic waves are employed. Pulsed acoustic waves such as ultrasound pulse waves and shock waves are used e.g. for the destruction of urinary stones and gallstones (concrements), for pain treatment, and the treatment of obstructed vessels and the heart muscles, etc. Continues acoustic waves are used to warm up tissue, e.g. to destroy tumors.

The therapeutic acoustic wave source generally has a focus that is determined by its geometry. The focus can e.g. be created by a lens. The target location of the therapeutic acoustic wave source is defined as that point in space where the user, as indicated by the manufacturer, should position e.g. the concrements for treatment. The target location is typically the same as the focus location or very close to it. In the following, the word "focus" will be used as a synonym for the target location.

To visualize the concrements or the target, an imaging device is needed. Nowadays, either x-ray or ultrasound imaging is normally used.

For example, an image-forming ultrasound system provides two-dimensional sectional views of the body. If the position of the ultrasound transducer, which determines the image geometry, is known relative to the position of the therapeutic acoustic wave source, the position of the focus within the ultrasound image can be determined provided the focus lies within the image plane. If the therapeutic acoustic wave focus in the image is marked, e.g. as a graticule, the target e.g. the concrement, can be brought into alignment with the marking by means of an appropriate positioning device, thus ensuring that it is precisely the target that gets treated with the pressure pulses, e.g. the shockwaves.

An example of such a system is the lithotripter Dornier Delta II with isocentrical ultrasound localisation. There an ultrasound transducer is coupled to a pressure pulse source via a mechanical device according to DE 3915384 in such a way that the focus always remains within the center line of the 2D image plane by restricting the mechanical degrees of freedom between the pressure pulse source and the image-forming ultrasound transducer. In addition, the distance between the transducer surface and the focus is recorded by a sensor so that the position of the focus location in the 2D ultrasound image is known. This is marked by a graticule. The user can now align the target structure in the body with the shockwave focus by moving the patient by means of a patient table under ultrasound control.

The superimposition of the graticule requires a signal input at the ultrasound scanner, a data communication protocol between the pressure pulse device and the ultrasound scanner and a special software version of the ultrasound scanner. This represents a relatively expensive solution, particularly when ultrasound scanners of different designs have to operate with the shockwave therapy device.

EP 1882452 describes a treatment instrument, that is a shockwave source, as well as an image forming device such as for example, an ultrasound transducer for generating an image of the treatment region. This image from the image forming device represents a first signal that is transferred from the image forming device to a display monitor via a control unit. Further, a second signal generated by the treatment instrument, a so-called location or orientation signal is also relayed via the control unit to the display monitor. This signal represents the position of the focal volume of the treatment instrument with respect to the image forming device.

The display monitor thus receives two different image signals by means of which it displays the image of the region to be treated and superimposes thereon the focal region of the treatment instrument. However, this document does not advise how the relative position of the two different image signals that are superimposed on the display monitor is determined.

DE 195 19 956 A1 discloses a method to calibrate the device before starting the treatment to ensure that the focal region of the acoustic wave source matches with a position on a display image of an x-ray image unit. A ring with an x-ray positive marking is brought in the optical path of the x-ray unit. The spatial position of the ring is determined via a sensor system fixed to the ring. The marking includes a scale of length and angles. The treatment region and the scale are visualized so that the position of the treatment region can be measured. The position of the treatment region on the monitor is marked by a mouse click on the screen.

This calibration does not allow the user to change image depth, zoom or to mirror the image once it is calibrated. Furthermore, the calibration needs extra time.

A different approach is disclosed in patent specification DE 195 48 000, describing a method and an apparatus with the features of the preamble of claims 1 and 28. There, a sensor/transmitter unit is disclosed generating an additional ultrasound signal that is transmitted in such a way that it is processed by the ultrasound transducer as a tissue echo. In order that this signal should appear at the right place in the image, the sensor must register the moment when the ultrasound transducer generates a particular scan line. The disadvantage of this solution is that the sensor must be positioned between the patient and the ultrasound transducer by a mechanical coupling to the therapy device, thus hindering treatment.

It is therefore the object of the invention to improve a method as well as an apparatus of the above-mentioned type so that the ultrasound unit and the pressure pulse device can be coupled and cooperate with each other in an easy and flexible way.

According to the invention, this object is achieved by a method for assigning a marking indicating a focus of a therapeutic acoustic wave source to a position on an ultrasound image having the features of claim 1.

In providing a separate correlation unit, the invention permits the data of the therapeutic acoustic wave source and the image data of the ultrasound unit to be processed separately, and thus rather independently from the ultrasound unit. The invention provides a different approach by processing and modifying the image coming from the ultrasound unit in a separate correlation unit before this modified display image is transmitted from the correlation unit to a display element like a monitor. This means that the correlation unit receives conventional standard output data from the ultrasound unit, namely the image, and from the therapeutic wave device, namely, the position of the therapeutic acoustic wave source or its focus with respect to the ultrasound transducer of the ultrasound unit. These data are processed within the correlation unit 4 rather independently from their format or from data communication protocols of the devices generating these data.

The correlation actually takes the information from the display image itself and assigns a focus marking to a correlated position on the ultrasound image. This decreases the need for costly technical modifications of the image forming modality, namely the ultrasound unit itself. The invention can therefore be used with many different kinds of therapeutic acoustic wave devices and ultrasound units, thus increasing compatibility of these devices. As the correlation unit can receive regular standard data sent from common therapeutic acoustic wave devices and ultrasound devices, it is also easy to use.

In an embodiment of the invention, the display image is read in matrix form. Thereby, a data format independent from the ultrasound unit is achieved.

Advantageously, the relative position of the therapeutic acoustic wave focus can be transformed into coordinates of the ultrasound coordinate system and presented to the correlation unit. Thus, the coordinates of the focus in an ultrasound coordinate system are known.

Additionally, assigning the focus marking can be accomplished by transformation of the focus coordinates known in the ultrasound coordinate system into matrix coordinates. With the information given to the correlation unit, namely the display image and the focus coordinates in the ultrasound coordinate system, this can be accomplished rather independently from the pressure pulse therapeutic acoustic wave device and the ultrasound unit and from any further information therefrom.

Favourably, the display image matrix can be segmented, thereby dividing the display image matrix into sub-areas representing an ultrasound image and further image information, like a depth scale.

In a further embodiment of the invention, the position of the ultrasound image within the display image matrix is determined by segmentation. This forms the basis for further calculations and for the assignment of the focus marking.

In addition, a depth scale can be extracted from the display image matrix by segmentation, said depth scale permitting correlation of matrix elements with the coordinates in the ultrasound coordinate system as well as calculation of a scaling factor of the ultrasound image.

In a variant of the invention, a horizontal scale can be extracted from the display image matrix by segmentation. This enables to determine the y- location of the x-axis, e.g. an axis of the ultrasound coordinate system, directly from the display image matrix.

In an advantageous embodiment of the invention, segmentation can be accomplished by means of a threshold value method, the accuracy of which is easily adjustable.

Further, the depth information can be determined from recognized labels of the depth scale. Thereby, determining the actual depth value of a specific matrix element is possible, independent from the scaling of the ultrasound image.

Alternatively, the depth information can be determined by counting the graduation marks on the depth scale having a known spacing, thereby also allowing to determine the actual depth value of a specific matrix element from the display image as such, decreasing or even eliminating the need for further information from the ultrasound unit.

Favourably, the orientation of the ultrasound image can be determined directly from the ultrasound image data within the display image matrix (23), thereby decreasing or even eliminating the need for further information or data from the ultrasound unit, rendering the inventive method rather independent therefrom.

In a further embodiment of the invention, a vertical orientation of the ultrasound image can be determined by calculating the vertical centre of gravity of the ultrasound image. Thus, also the vertical orientation of the ultrasound image can be determined independently from the ultrasound unit.

Favourably, a vertical orientation of the ultrasound image can be identified by determining the location of the point of intersection of the extension of the left and right lateral borders of the ultrasound image. This allows to determine the orientation on the basis of the display image as such, rather independently from additional information from the ultrasound unit.

Additionally, the x- location of the y-axis of the ultrasound coordinate system can be identified by determining the horizontal centre of gravity of the ultrasound image. Thus, the x- location of the y-axis can also be achieved directly from the ultrasound image and thus independently from further information from the ultrasound unit.

Further, the y- location of the x-axis of the ultrasound coordinate system can be determined directly from the display image matrix, and thus independently from further information or data from the ultrasound unit.

Favourably, a scaling factor of the ultrasound image can be determined directly from the display image matrix, and thus also rather independently from the ultrasound unit.

In a variant of the invention, at least one arbitrary y-coordinate of the display image matrix can be correlated with a y-value of the y-axis of the ultrasound coordinate system by means of the depth scale. Thus, correlation of the ultrasound coordinate system with the display image matrix can be accomplished directly by means of the display image matrix itself, rather independently from any further information from the ultrasound unit.

In addition, a scaling factor of the ultrasound image can be determined by comparison of a distance between two graduation marks of the depth scale in the display image with an actual distance given by the labels of the depth scale corresponding to these two graduation marks. Thus, also the scaling factor can be determined by the correlation unit directly from the display image matrix as such, and thus rather independently from further information from the ultrasound unit.

In a favourable embodiment of the invention, a scaling factor of the ultrasound image can be determined by a comparison of a known distance between two adjacent graduation marks of the depth scale with a measured distance between said graduation marks. This allows to determine a scaling factor of the ultrasound image on the basis of the display image as such, thereby rather eliminating the need for further information from the ultrasound unit.

Advantageously, the horizontal orientation of the ultrasound image can be determined by identifying a left/right identification mark in the display image. This allows a quick, easy and good determination of the horizontal orientation of the ultrasound image and thus of a horizontal orientation factor of the ultrasound image from the display image.

Additionally, the matrix coordinates of the focus can be calculated from the focus coordinates in the ultrasound coordinate system, the origin of the ultrasound coordinate system, i.e. y- location of the x-axis and the x- location of the y-axis, the orientation factors and the scaling factor. Thus, the whole procedure of calculating the matrix coordinates of the focus can be accomplished directly on the basis of the input data of the correlation unit, namely the display image and the focus coordinates in an ultrasound coordinate system, rather independent from further information from the ultrasound unit.

Further, if calculation of the position of the focus marking fails at some step, an error message can be superimposed on the display image, thereby increasing the safety of the method and the whole therapeutic acoustic wave treatment.

In an advantageous embodiment of the invention, if the focus does not lie within a certain sector plane scanned by the ultrasound transducer, but within the plane spanned by said sector plane, a corresponding message can be superimposed on the display image. This gives the user or a physician additional information about the actual location of the focus and thus allows or facilitates for the physician a quicker correction of the focus position or ultrasound scanner settings.

Moreover, the superimposed message can comprise a marking indicating the location of the focus outside the sector plane, thereby giving even further information to help the physician to correct the position of the focus or ultrasound scanner settings, that is, aligning the focus with the scanned sector plane. This facilitates the handling for the physician and accelerates the treatment procedure.

In a favourable embodiment of the invention, the superimposed message comprises a marking indicating in which direction the focus has to be moved in order to align it with the scanned sector plane. This further increases the additional information the physician gets about the actual location of the focus and thus allows a good and quick aligning of the focus with the scanned sector plane.

Additionally, if the focus does not lie within a plane spanned by the sector plane scanned by the ultrasound transducer, a corresponding message can be superimposed on the display image comprising a marking indicating the point of intersection between a focus axis and said sector plane. This allows a good and quick correction of the depth of the focus or the position of the ultrasound transducer so that the focus aligns with the sector plane.

Favourably, an indicator in the display image showing if the display image is live or frozen can be detected and a corresponding message can be superimposed on the display image, thereby further increasing the safety of the method and the whole therapeutic acoustic wave treatment.

The object is further attained with the features of claim 12.

Providing the inventive separate correlation unit between the therapeutic acoustic wave device, the ultrasound unit and the display unit allows the processing of data coming from the therapeutic acoustic wave source and the ultrasound unit rather independent from the ultrasound unit, that is, independent from further data or information processed in the ultrasound unit and software programs or data protocols of the ultrasound unit. As the correlation unit is able to determine a lot of information needed for calculating the focus in matrix coordinates from the display image as such in correlation with the focus coordinates in an ultrasound coordinate system, that is the input data being necessary to be presented to the inventive correlation unit are very basic data, the correlation unit is compatible to a lot of commonly used ultrasound units and therapeutic acoustic wave sources. It thus operates as an easy to handle, interface device for connecting different therapeutic acoustic wave devices with different ultrasound units, thereby increasing cooperation possibilities and overcoming compatibility problems of these devices.

In an embodiment of the invention, the correlation unit can further comprise a read-in unit for reading in the display image in a matrix format, thereby permitting the display image to be divided into an array of single matrix elements and transformed into a neutral format independent from the ultrasound unit.

Further, the correlation unit can comprise an image recognition unit for segmentation of the display image matrix, allowing recognition of certain segments, namely further information like a depth scale contained in the display image.

Advantageously, the segments recognized by the image recognition unit can comprise the outline of the ultrasound image. This permits recognition of the actual ultrasound image. Depending on the shape of the outline, this may also allow the drawing of conclusions about the location and orientation of the ultrasound image.

Thus, the actual ultrasound image and its position within the display image matrix are known. The segments recognized by the image recognition unit may comprise the set of matrix elements belonging to the ultrasound image. Depending on the spatial distribution of the matrix elements, this may also allow the drawing of conclusions about the location and orientation of the ultrasound image.

Favourably, the segments recognized by the image recognition unit can comprise a depth scale contained in the display image matrix, allowing determining information about the actual depth directly from the display image matrix.

In a further embodiment of the invention, the segments recognized by the image recognition unit can comprise labels of the depth scale, indicating specific depth values.

Additionally, the segments recognized by the image recognition unit can comprise a horizontal scale. This allows to also determine the y- location of the x-axis, e.g. an axis of the ultrasound coordinate system, directly from the display image matrix and thus independent from further information from the ultrasound unit.

In a favourable embodiment of the invention, the correlation unit can further comprise a vertical scale recognition unit for reading the information from the display image matrix, allowing written information contained in the display image matrix to be recognized and to be read out.

Further, the information can comprise depth information contained in a labelling of a depth scale. This permits determining the actual depth of an ultrasound image point that is a matrix element, being part of the ultrasound image and also serves as a basis for determining the scaling of the ultrasound image.

In addition, the information can comprise the unit of the depth scale, which helps to interpret the depth information gathered from the labels of the depth scale correctly.

Favourably, the vertical scale recognition unit is capable of determining a scaling factor of the ultrasound image, independent from further information from the ultrasound unit.

In an embodiment of the invention, the scaling factor can be a ratio of the depth distance given by at least two depth scale labels and the actual distance between two graduation marks on the depth scale corresponding to said labels. The factor can thus be determined directly from the display image matrix independent from further data from the ultrasound unit.

In addition, the vertical scale orientation unit can be further capable of identifying the vertical orientation of the ultrasound image, permitting to determine the orientation of the ultrasound image within the display image matrix rather independent from further information from the ultrasound unit.

Additionally, the correlation unit can further comprise a horizontal orientation unit for identifying the horizontal orientation of the ultrasound image, thereby also allowing to determine the horizontal orientation of the ultrasound image rather independently from further information of the ultrasound unit, but on the basis of the ultrasound image as such.

In a variant of the invention, the vertical scale orientation unit can be further capable of correlating a matrix row with a y-value of the y-axis of the ultrasound coordinate system by means of the depth scale. Thereby, allowing correlation of the matrix elements with coordinates in the ultrasound coordinate system directly on the basis of the display image matrix itself, and thus independent from further information from the ultrasound unit.

Favourably, the correlation unit can further comprise a calculating unit for calculating the position of the focus in the display image matrix on the basis of the focus coordinates in the ultrasound coordinate system given to it. This allows the coordinates of the pressure pulse source focus to be determined in matrix coordinates directly from the input data of the correlation unit, independent from further information from the ultrasound unit.

In an embodiment of the invention, a y-matrix coordinate of the focus can be calculated on the basis of the vertical orientation factor, the scaling factor, the correlation between a matrix row and a y-value on the y-axis of the ultrasound coordinate system and the y-coordinate of the focus in the ultrasound coordinate system transmitted to the correlation unit. The y-matrix coordinate of the focus thus being calculable solely from information and data derived from the display image as such, and the y-coordinate of the focus in the ultrasound coordinate system, thereby decreasing or eliminating the further information needed from the ultrasound unit.

Further, an x-matrix coordinate of the focus can be calculated on the basis of the centre line of the ultrasound image, the horizontal orientation factor, the scaling factor, and the x-coordinate of the focus in the ultrasound coordinate system transmitted to the correlation unit. Thereby, permitting also the x-matrix coordinate of the focus to be calculated independently from further information from the ultrasound unit.

Additionally, the correlation unit can further comprise a marking unit for marking the display matrix coordinates corresponding to the focus, allowing the end user to easily identify the focus on the ultrasound image.

In a further embodiment of the invention, the correlation unit can further comprise an output unit for transmitting the display image matrix with a focus marking thereon to a display unit. Thus, the correlation unit can be connected to common display elements like a monitor, thereby facilitating the use and handling of the inventive correlation unit.

In the following, an embodiment of the invention is described based on the following drawing.

Figure 1      shows an arrangement of a therapeutic acoustic wave device, an ultrasound unit and a display element, according to the prior art,

Figure 2      shows the inventive correlation unit being arranged between the components shown in Figure 1,

Figure 3      shows a schematic layout of the inventive correlation unit,

Figures 4 a to c      show a therapeutic acoustic wave source and an ultrasound transducer of an ultrasound unit arranged with respect to each other,

Figure 5      shows a display image of the ultrasound unit,

Figure 6      shows the display image of Figure 5 converted into a matrix format,

Figure 7      shows the display image matrix of Figure 6 with a coordinate system of the ultrasound transducer displayed therein,

Figure 8      shows the display image matrix of Figure 7 with y-values or rows of the matrix being correlated with a y-value of the y-axis of the ultrasound coordinate system of the ultrasound transducer,

Figure 9      shows the display image with the borders of the ultrasound image being extended until they intersect,

Figure 10      shows a focus of a therapeutic acoustic wave source being marked in the display image matrix of Figure 8, and

Figure 11      shows a marking indicating the position of the focus on the display image matrix.

The present invention basically relates to a method and apparatus to be used in therapeutic acoustic wave treatment. In this application, the term "therapeutic acoustic wave" refers to different kinds of therapeutic acoustic wave and therapeutic acoustic wave devices. The term for example also includes pressure pulses, shockwaves, high intensity focused ultrasound (HIFU), focused ultrasound surgery (FUS) and other acoustic waves.

For the purpose of explanation and for the sake of clarity, the following specific embodiment of the invention is described with reference to a shockwave device 1 and to shockwaves. Nevertheless, the functional principles and the design of the inventive correlation unit 4 are the same for applications using other kinds of therapeutic acoustic waves.

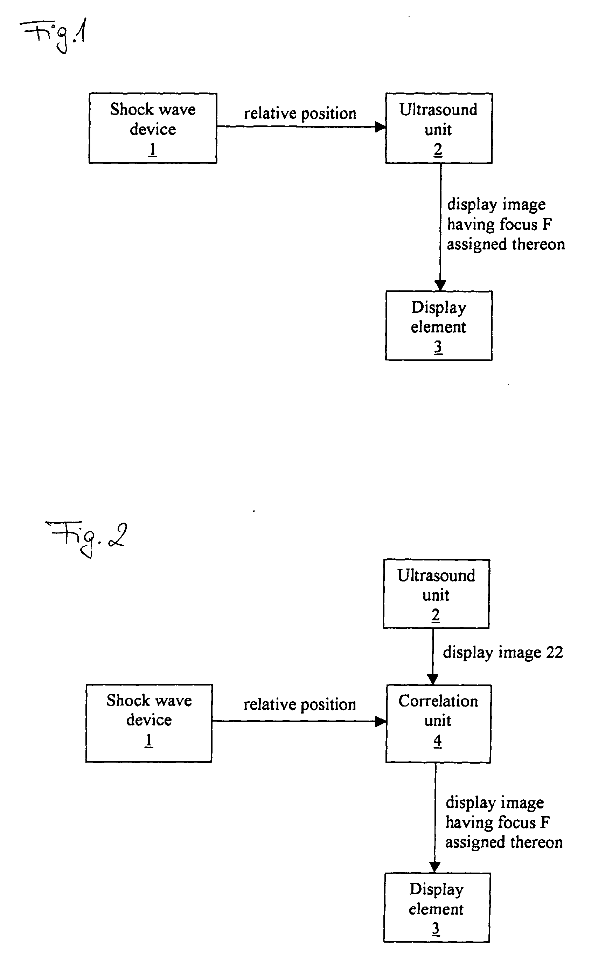

Figure 1 shows an arrangement of a shockwave device 1, an ultrasound unit 2 and a display element 3, like a monitor, for displaying the ultrasound image generated by the ultrasound unit as well as a marking indicating a focus F of the shockwave device 1 being superimposed on the ultrasound image, according to the prior art.

Here, the shockwave source of the shockwave device 1 is guided such that its focus always lies within the ultrasound image plane of the ultrasound transducer by means of a compulsory guide. Additionally, the distance between the transducer surface and the focus of the lithotripter is recorded by a sensor which in this embodiment of the invention belongs to the shockwave device 1. Alternatively, the shockwave device 1 is provided with some kind of electromagnetic, acoustical or optical tracking system detecting the position of the shockwave source of the shockwave device 1 with respect to an ultrasound transducer of the ultrasound unit 2. This allows the position of the focus F known in a shockwave coordinate system to be transformed into a position in an ultrasound coordinate system and thus to be assigned to a corresponding position on an ultrasound image generated by the ultrasound transducer, which can then be presented by a display element 3, like a monitor, as indicated in Figure 1.

However, in this prior art technique, the data format, the software system and/or the data protocol used to process the data within the ultrasound unit have to be known, and the ultrasound unit has to be adapted to the shockwave device in order to ensure a correct cooperation of these devices.

The invention, in contrast, permits correlating and thus processing, image data of the ultrasound unit 2 and data of the shockwave device 1 in a separate correlation unit 4, thereby decreasing the effort for directly adapting the ultrasound unit to the shockwave device and thus allowing different ultrasound units to be connected to different shockwave devices by the inventive correlation unit 4.

Therefore, the correlation unit 4 is arranged between the shockwave device 1, the ultrasound unit 2 and the display element 3, like for example a monitor, as shown in Figure 2. The same reference numerals as used in Figure 1 are used for designating the corresponding parts in Figure 2, and the description of those elements is omitted.

Analogously, as described above, the position of the focus F of the shockwave source of the shockwave device 1 in relation to the ultrasound transducer of the ultrasound unit 2 is detected and transformed into a position in the ultrasound coordinate system. In the present embodiment, this transformed focus position $(\tilde{x}_F, \tilde{y}_F)$ is transmitted to the correlation unit 4 from the shockwave device 1.

Alternatively, in a different embodiment of the invention, this coordinate transformation of the spatial position of the focus into a focus position $(\tilde{x}_F, \tilde{y}_F)$ in an ultrasound coordinate system can also be processed directly within the correlation unit 4.

For example, if a tracking system with cameras is used for detecting the relative position of the shockwave source and the ultrasound transducer. In this case, both the ultrasound transducer and the shockwave source can be provided with markers detected by one or more cameras. The information of the position of the shockwave source and thus its focus with respect to the ultrasound transducer can be transmitted to the correlation unit 4, which then transforms the spatial position of the focus with respect to the transducer into a focus position within the ultrasound coordinate system.

Also, a display image, which in the prior art would be directly sent from the ultrasound unit 2 to a display element 3, is transmitted from the ultrasound unit 2 to the correlation unit 4. The inventive correlation unit 4 permits achieving the same result as the prior art, namely displaying the ultrasound image with a focus marking assigned to a correlated position thereon, by processing these two input data.

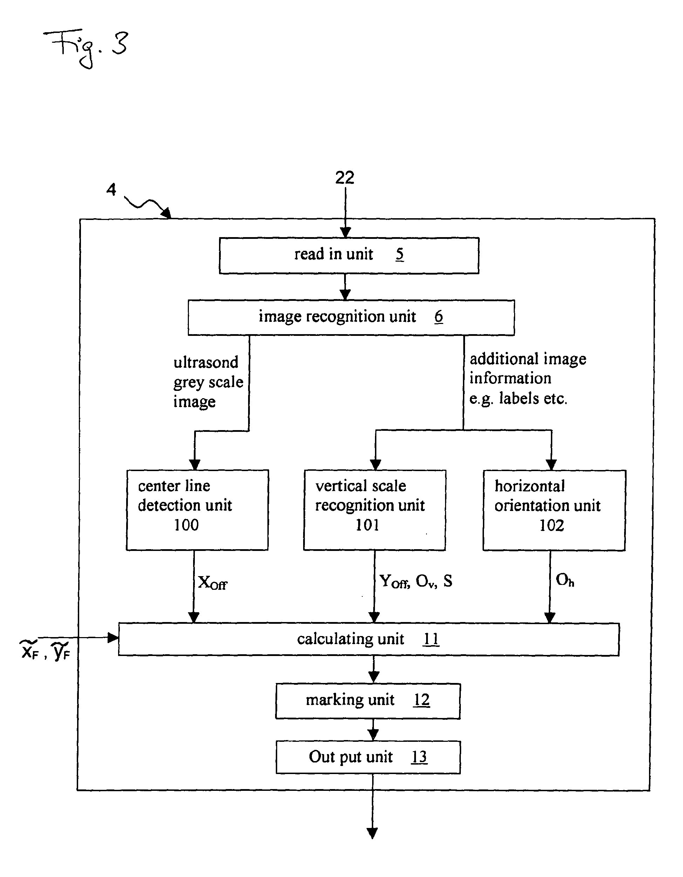

Figure 3 schematically shows the layout of the inventive correlation unit 4. The reference numerals used in Figure 3 indicate the same parts as in Figures 1 and 2, so that reference is made to the description of Figures 1 and 2 in this regard.

The correlation unit 4 of the present embodiment comprises a read-in unit 5 for reading in the display image 22 sent from the ultrasound unit 2 in a matrix format, an image segmentation unit 6 for identifying the actual ultrasound image 17 within the display image matrix 23 and for identifying possible further symbols or elements 21, 24 therein as well as written information in the display image matrix 23.

Further, the correlation unit 4 comprises a center line detection unit 100 for determining a center line of the ultrasound image 17, a vertical scale recognition unit 101 for determining a scaling factor of the ultrasound image 17 in correlation to the display image in matrix format, a vertical orientation factor of the ultrasound image 17 and the origin of the y axis of the ultrasound coordinate system 18 as well as a horizontal orientation unit 102 for determining a horizontal orientation of the ultrasound image 17.

Additionally, the correlation unit comprises a calculating unit 11 for determining a matrix element corresponding to the coordinates of the focus F in the ultrasound coordinate system.

Finally, the correlation unit 4 of the present embodiment comprises a marking unit 12 for making the specific determined matrix element and/or other matrix elements in the vicinity thereof, as well as an output unit 13 for sending data to a further processing unit, e.g. a display element 3 like a monitor.

Although the correlation unit 4 is described as a separate unit, this unit does not necessarily have to be located in a separate housing. In alternative embodiments of the invention, the correlation unit 4 can also be integrated into already existing devices such as, for example, into the shockwave device 1.

In a further alternative embodiment of the invention, the correlation unit 4 could, for example, be arranged or located on a computer system, to which also the shockwave device 1 and the ultrasound device 2 are connected.

In the following, the mode of operation of the inventive embodiment shown in Figures 2 and 3 will be described.

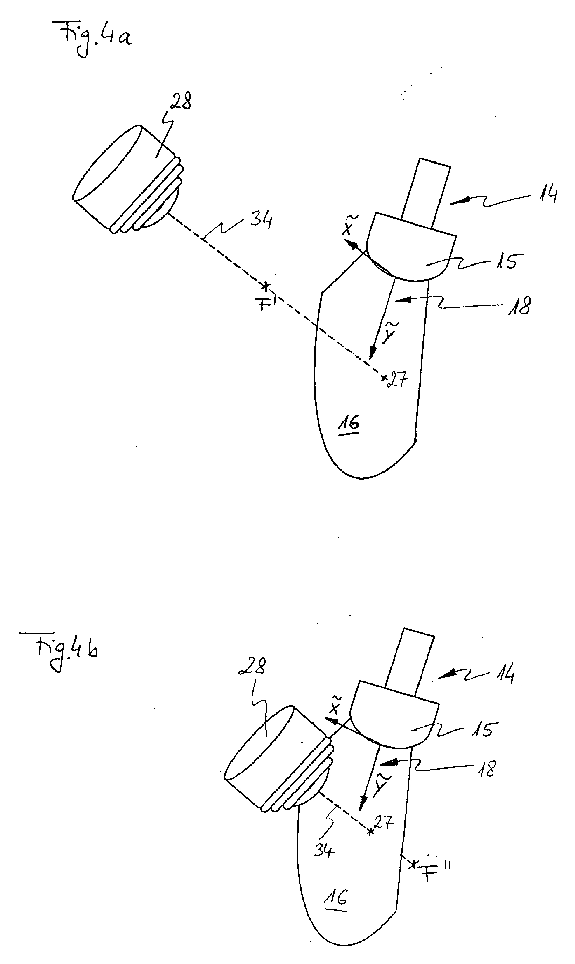

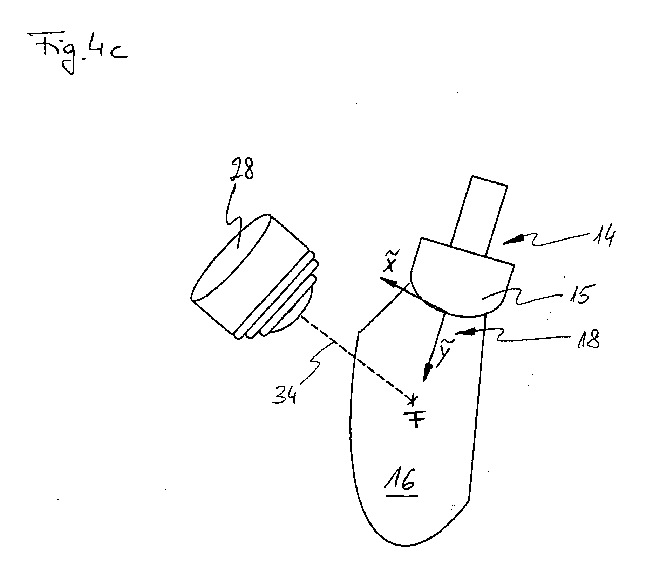

Figures 4 a to c show a shockwave source 28 of a shockwave device 1 and an ultrasound image transducer 14 of an ultrasound unit 2 arranged with respect to each other. The reference numerals used in Figures 4 a to c indicate the same parts as in Figures 2 and 3, so that reference is made to the description of Figures 2 and 3 in this regard.

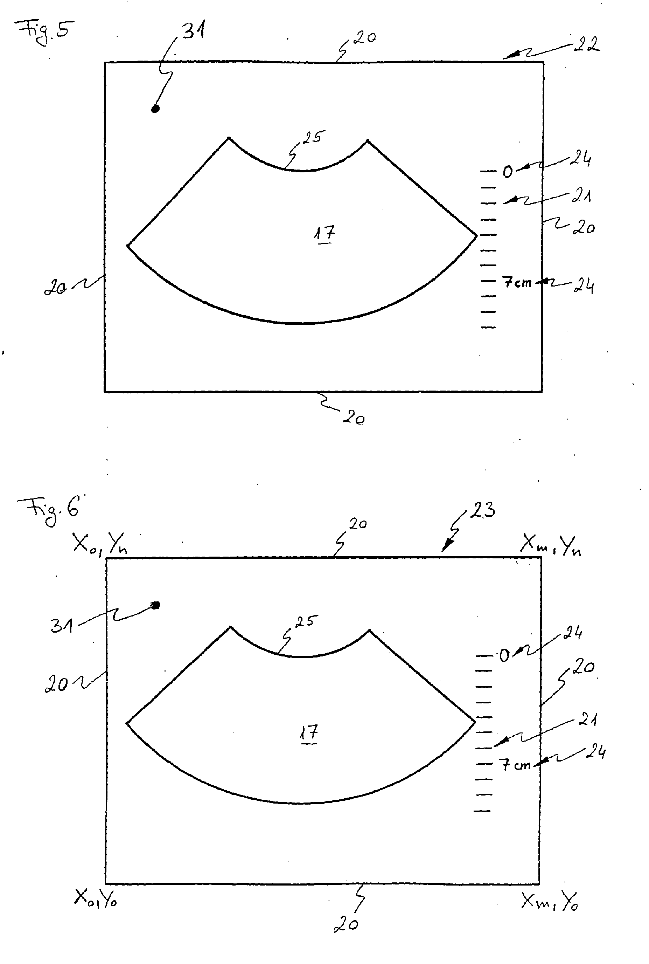

The ultrasound transducer 14 having a head 15, which emits and receives ultrasound waves, scans a sector plane 16 e.g. of a patient's body, and generates an ultrasound image 17 (see Figure 5) corresponding to the scanned plane 16. Depending on the position of the shockwave source 28 in relation to the ultrasound transducer 14, a focus F of the shockwave source 28 can be located on either side of the two-dimensional sector plane 16 scanned by the ultrasound transducer 14, as indicated by reference signs F' and F" in Figures 4a and b. This means the focus of the shockwave source 28 can be in front of the sector plane 16, as shown in Figure 4a in which the focus is indicated F'. Alternatively, the focus of the shockwave source 28 can be, however, located behind the sector plane 16 of the ultrasound transducer 14 as shown in Figure 4b in which the focus is indicated F".

However, in the present embodiment of the invention, and for explanation of the inventive method for assigning a marking indicating a focus F of a shockwave source 28 to a position on an ultrasound image below, it is supposed that the focus F of the shockwave source 28 lies within the sector plane 16 scanned by the ultrasound transducer 14, as shown in Figure 4c. In practice, this can be accomplished by providing a compulsory guide, guiding the movements of the shockwave source 28 and the ultrasound transducer 14 such that the focus F always lies within the sector plane 16.

It is further supposed that in the present embodiment of the invention, the head 15 of the ultrasound transducer 14 is a sector ultrasound transducer, as most commonly used in practice nowadays, generating a sector ultrasound image 17, as shown in Figure 5.

It is supposed that the sector plane 16 is two-dimensional. Therefore, an ultrasound coordinate system having axis $\tilde{x}, \tilde{y}$ (indicated 18) can be defined which contains the sector plane 16. Without loss of generality, the origin of this ultrasound coordinate system 18 coincides with the point of intersection of transducer and center scan line. By means of a tracking system or the like the position of the focus F with respect to the ultrasound transducer is known as described with regard to Figures 1 and 2. Hence, the coordinates $(\tilde{x}_F, \tilde{y}_F)$ of the focus F lying within the sector plane 16 can be determined in the ultrasound coordinate system 18, by coordinate transformation.

Figure 5 shows a display image 22 as it is generated by an ultrasound unit 2 for transmission to a display element 3. The reference numerals used in Figure 5 indicate the same parts as in Figures 2 to 4, so that reference is made to the description of Figures 2 to 4 in this regard. The ultrasound transducer 14 generates a sector shaped ultrasound image 17, as the one shown in Figure 5. This image can usually be enlarged or reduced as well as mirrored for example on the axis of the ultrasound coordinate system 18 by the user according to his demands.

In order to fill a whole square or rectangular display element 3, like a monitor, the remaining space between the sector ultrasound image 17 and the display edges 20 is usually filled with a unitary colour, for example a deep gray or black. Often, also a depth scale 21, the name of the patient or one or both axes of the ultrasound coordinate system 18 are also integrated in this display image 22. However, in the present embodiment, only a labelled depth scale 21 is present, as shown in Figure 5. According to the inventive method, this square display image 22 is transmitted to the correlation unit 4.

The task of the correlation unit 4 generally is as follows. The read-in unit 5 of the correlation unit 4 receives the display image 22 and reads it into a matrix format, resulting in a matrix 23 having m elements in the x-direction and having n-elements in the y-direction, as shown in Figure 6. The reference numerals used in Figure 6 indicate the same parts as in Figures 2 to 5, so that reference is made to the description of Figures 2 to 5 in this regard. Thus, each element of the matrix 23 has unique matrix coordinates $(X_i, Y_j)$. In Figure 6, the matrix coordinates of the vertices of the display image matrix 23 are given. An important function of the correlation unit 4 is to relate a correct matrix element $(X_F, Y_F)$ to the focus coordinates $(\tilde{x}_F, \tilde{y}_F)$. If the shockwave focus F is contained within the sector plane 16, there has to be a corresponding matrix element within the ultrasound image 17 presented within the display image matrix 23. The axes $(\tilde{x}, \tilde{y})$ define a coordinate system 18, which includes the sector scan plane 16. There exists a coordinate transformation, i.e. there are functions f and g with:

$$X = f(\tilde{x}, \tilde{y}), \qquad \text{Eq. 1a)}$$

$$Y = g(\tilde{x}, \tilde{y}). \qquad \text{Eq. 1b)}$$

$X, Y$ is a coordinate system, which includes the ultrasound image 17. The functional relationship, which is:

$$X = O_h S \tilde{x} + X_{off} \text{ with } X_{off} = X(\tilde{x} = 0) \qquad \text{Eq. 2a)}$$

$$Y = O_v S \widetilde{y} + Y_{off} \quad \text{with} \quad Y_{off} = Y(\widetilde{y} = 0) \qquad \text{Eq. 2b)}$$

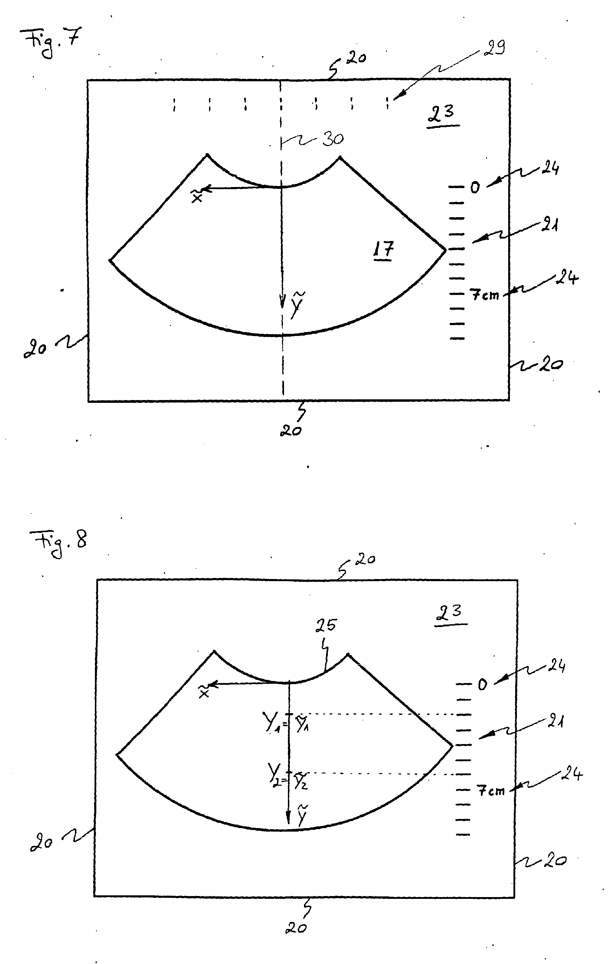

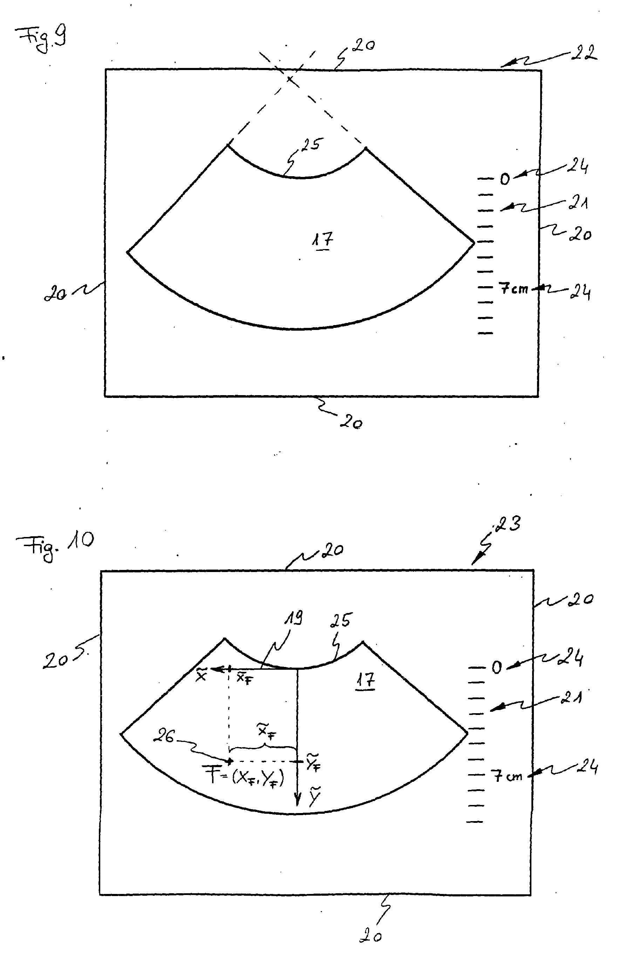

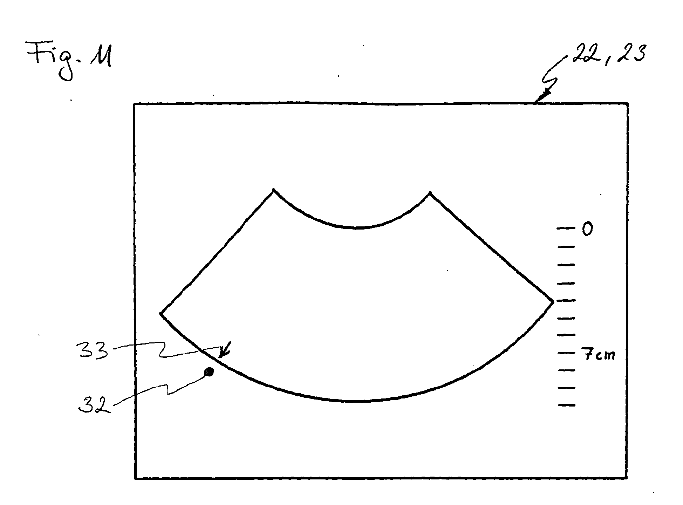

$(X_{off}, Y_{off})$ is the matrix element of the transformed origin of the ultrasound coordinate system $(\widetilde{x}, \widetilde{y})$, i.e. its zero point. The coordinate $(\widetilde{x}, \widetilde{y})$ is depicted in Figures 7, 8 and 10 as being displayed in the display image. Nevertheless, its x- and y- axes do not necessarily need to be displayed in the display image, as for example shown in Figures 9 and 11.

$S$ is a scaling factor ($S \geq 0$). $O_h$ and $O_v$ define the orientation of the axes. In the present embodiment, the horizontal orientation $O_h$ is -1 for a leftward directed ultrasound image 17 and +1 for a rightward directed ultrasound image 17. The vertical orientation $O_v$ is +1 for an upwardly directed ultrasound image 17 and -1 for a downwardly directed ultrasound image 17.

In order to relate the correct matrix element $(X_F, Y_F)$ to the focus coordinates $(\widetilde{x}_F, \widetilde{y}_F)$, the values $O_h, O_v, S, X_{off}$, and $Y_{off}$ have to be determined. This process is described in more detail below.

The image read in into a matrix format by the read-in unit 5 is then further processed by the image recognition unit 6 of the correlation unit 4. The image recognition unit 6 detects the actual ultrasound image 17 within the display image matrix 23. Thus, also the position of the actual ultrasound image 17 in the display image matrix 23 is known. Further, the image recognition unit 6 can also identify a scale, like a depth scale 21, and further elements possibly contained in the display image matrix 23, like the labelling 24 of the scale 21, other writing or a horizontal scale in the display image matrix 23. This can, for example, be accomplished by segmenting the display image matrix 23 using a threshold value method. The ultrasound image 17, containing mostly gray values, can be distinguished from the remaining area of the display image, which usually is black, and from the writing, the depth scale as well as graduation marks and labels thereof, which usually are white. However, in different embodiments of the invention, these colours may also vary.

The determination of the parameters $O_h$, $O_v$, $S$, $X_{off}$ and $Y_{off}$ is demonstrated for different examples in a stepwise manner.

In a first step, the parameter $X_{off}$ is deduced. In this embodiment of the invention, the ultrasound transducer 14 generating the ultrasound image 17 is supposed to be a sector transducer generating an ultrasound image 17 having a particular shape and contour, as shown in Figures 5 and 6. Segmentation of the ultrasound image 17 is equivalent to the determination of a set of $l$ display image matrix 23 elements $(X_i, Y_i)$, $i = 1,...,l$ belonging to the ultrasound image 17.

The vertical center line of the ultrasound image 17 is determined in matrix coordinates by center line detection unit 100 of correlation unit 4. One possible solution is to employ a calculation of the center line 30 of the ultrasound image area:

$$X_{center\ line} = \sum_{i=0}^{l} \frac{X_i}{l} \qquad \text{Eq. 3)}$$

as shown in Figure 7. The reference signs used in Figure 7 indicate the same parts as in Figure 2 to 6, so that, in this regard, reference is made to the description of Figures 2 to 6.

According to the above definition of the coordinate system $\widetilde{x}, \widetilde{y}$, this center line 30 of the sector ultrasound image 17 corresponds to the $\widetilde{y}$ axis. Therefore, $X_{off} = X_{center\ line}$.

Alternatively, assuming there is a horizontal scale 29 presented as ticks, as indicated by the dashed lines in Figure 7, the horizontal positions of the ticks can be determined by standard means of image processing and pattern recognition as, for example, by a threshold value method. The horizontal position of the center tick gives $X_{off}$.

In a second step, the parameters $O_v$, $S$, and $Y_{off}$ are deduced by a vertical scale recognition unit 101 of the correlation unit 4. Assuming there is a vertical depth scale 21 with the labels 24, for example 0 and 7 cm, displayed, as shown in Figures 5 to 7, then by standard means of image processing and pattern recognition, there can be two different matrix lines $Y_1, Y_2$ determined, which can be correlated with Eq. 2 as follows, with two different $\widetilde{y}$ values $\widetilde{y}_1, \widetilde{y}_2$, as shown in Figure 8.

$$Y_1 = O_v S \widetilde{y}_1 + Y_{off} \qquad \text{Eq. 4a)}$$

$$Y_2 = O_v S \tilde{y}_2 + Y_{off} \qquad \text{Eq. 4b)}$$

The reference signs used in Figure 8 indicate the same parts as the reference signs used in Figures 2 to 7, so that reference is made to the description of Figures 2 to 7 in this regard.

If e.g. $\tilde{y}_1 < \tilde{y}_2$, then according to our definition of the vertical orientation, $O_v$ is +1, in case $Y_1 < Y_2$ (upwardly directed image) and $O_v$ is -1, in case $Y_1 > Y_2$ (downwardly directed image). Inserting $O_v$, the two equations determine the scaling factor $S$ and $Y_{off}$.

Alternatively, assuming there is a vertical scale presented as ticks with known spacing and offset, but no labels. The vertical positions of the ticks can be determined by standard means of image processing and pattern recognition. From the difference between two neighbouring ticks, the scaling factor $S$ can be determined. The vertical orientation $O_v$ of the image may be determined by the contour of ultrasound image 17. Lines may be fitted to the left and right lateral image borders, as shown in Figure 9. If the extended lines intersect below the image, it is upwardly directed, otherwise it is downwardly directed. Assuming a downwardly directed ultrasound image 17, as shown in Figures 5 to 9, the position of the highest vertical tick of the vertical scale 21 determines the offset value $Y_{off}$.

The reference signs used in Figure 9 indicate the same parts as the reference signs used in Figures 2 to 9, so that reference is made to the description of Figures 2 to 9 in this regard.

In a third step, the horizontal orientation $O_h$ is determined by horizontal orientation unit 102 of correlation unit 4. It is common practice that the left/right orientation of the ultrasound image 17 is indicated by a mark 31 on one side of the ultrasound image 17, as shown in Figures 5 and 6. By standard means of image processing and pattern recognition, the left or right position of the mark can be determined, which gives the orientation $O_h$. In case the focus is along the center line 30, this third step can be omitted.

If the calculation at one step fails, e.g. due to a not intended use of the ultrasound unit, such that the calculation of the focus position is not possible, the correlation unit 4 gives an error image superimposed on the display image as output.

Another practical problem appears; though the focus is contained in the extended scan plane spanned by sector plane 16, but not in the selected ultrasound image, namely in the delimited sector plane 16 as such, e.g. by an unsuitable zooming action of the user, the correlation unit 4 can give another error message superimposed on the display image as output.

Another problem may appear when the user freezes the image, e.g. for documentation and evaluation. The correlation unit can give another error message superimposed on the display image as output. In common ultrasound display images, there is also an indicator that shows if the image is frozen or a live image. This indicator can be detected in the same way the image orientation marker 31 is detected.

Finally, the focus of the shockwave source 28 can be determined in matrix coordinates by the calculating unit 11 of the correlation unit 4. Based on the information determined by the read-in unit 5, the image recognition unit 6, the center line detection unit 100, the vertical scale unit 101, the horizontal orientation unit 102 and the input coordinates of the focus $(\tilde{x}_F, \tilde{y}_F)$ in the ultrasound coordinate system (18), the calculating unit 11 can determine the focus matrix element $(X_F, Y_F)$ by insertion of $(\tilde{x}_F, \tilde{y}_F)$ into Eq.2: The matrix row $Y = Y_F$ of the focus in matrix coordinates from $Y_F = O_v S \tilde{y} + Y_{off}$. The matrix column $X = X_F$ of the focus and matrix coordinates can be obtained from $X_F = O_h S \tilde{x} + X_{off}$.

This information, namely the matrix coordinates $X_F$ and $Y_F$ of the focus are presented to the marking unit 12 of the correlation unit 4, which marks this position viz. the corresponding element of the display image matrix 23 and/or some surrounding matrix elements. This is accomplished by superimposing a marking 26, like a cross, at the above-determined position $X_F$, $Y_F$ of the display image matrix 23, as shown in Figure 10. The reference numerals used in Figure 10 indicate the same parts as in Figures 2 to 9, so that reference is made to the description of Figures 2 to 9 in this regard.

If, e.g. a cross with crossed lines having a length of 11 matrix elements or pixels is to be superimposed, as in the present embodiment, the pixels $(X_F-5, Y_F)$, $(X_F-4, Y_F)$ ... $(X_{F+}5, Y_F)$, and the pixels $(X_F, X_F-5)$, $(X_F, Y_F-4)$ ... $(X_F, Y_{F+}5)$ the display image matrix 23 are set to a value corresponding to the colour value of the marking 26. The display image matrix 23 is then passed to a display element 3, like a monitor, by output unit 13, for example, via a graphics card.

In different embodiments of the invention, the marking 26 can also have different shapes. For example, the marking 26 can be a smaller or larger cross as the one mentioned above. Alternatively, only the matrix element or pixel corresponding to the actual focus position $X_F$, $Y_F$ or a circle surrounding the actual focus position $X_F$, $Y_F$ having a smaller or larger radius can be marked.

If in alternative embodiments of the invention the focus F of the shockwave source 28 does not lie within the image plane, like in the described embodiment, e.g. because the ultrasound transducer 14 can be moved independently of the shockwave source 28, it can be useful to display the point of intersection 27 of the axial focus axis 34 with the image plane 16, as shown in Figures 4a and 4b. The procedure is analogous to that described above.

If, however, the focus F of the shockwave source 28 lies within the plane spanned by the sector plane 16 scanned by the ultrasound transducer 14 but is not part of the delimited sector plane 16 as such, it can be useful that in this case the correlation unit 4 outputs a message or superimposes a message image on the display image 22, 23 transmitted to the display element informing the user that the focus F lies outside the sector plane 16. In this context, also a marking could be superimposed on the display image indicating the location of the focus F. If the focus F lies to the left of the sector plane 16, the marking 32, for example a dot, can be displayed on the left border of the ultrasound image 17, as shown in Figure 11.

The reference signs used in Figure 11 indicate the same parts as the reference signs used in Figure 2 to 10 so that reference is made to the description of Figures 2 to 10 in this regard.

To provide even more support for the user, this first marking with the dot can be replaced or combined with another marking 33 indicating a direction as, for example, an arrow or a triangle, showing the user in which direction scanned sector plane 16 and thus the ultrasound transducer 14 has to be shifted in order to align the focus F with the sector plane 16, as shown in Figure 11. Alternatively, the marking, that is the arrow, can also indicate the direction in which the focus F is located.

Additionally, the marking 33 can also include a distance indicator. This could, for example, be accomplished by correlating the size or length of the marking, viz. the arrow, to the distance of the focus F from the sector plane 16.

Although the single steps of the above-described method for assigning a marking indicating a focus of a pressure pulse source to a position on an ultrasound image have been described in a specific order, it is clear to a person skilled in the art that the order of the single steps can be changed within a reasonable range without deviating from the present invention.

From the embodiment of the invention described above, it can be seen that the correlation unit 4, solely by means of the information contained in the display image itself, correlates the relative position of the focus to the ultrasound image and assigns a focus marking to a correlated position of the ultrasound image.

## Claims

1. Method for assigning a marking indicating a focus (F) of a therapeutic acoustic wave source (28) to a position on a display image (22) of an ultrasound unit (2) containing an ultrasound image (17),
**characterized in that,**
the display image (22) and the position of the focus (F) relative to an ultrasound transducer (15) of an ultrasound unit (2) are transmitted to a correlation unit (4) being separate from the ultrasound unit (2), which by means of the information contained in the display image (22) correlates the relative position of the focus (F) to the ultrasound image (17) and assigns a focus marking (26) to a correlated position on the ultrasound image (17).

2. Method according to claim 1,
**characterized in that,**
the display image (22) is read in a matrix form.

3. Method according to at least one of the preceding claims,
**characterized in that,**
the relative position of the therapeutic acoustic wave focus (F) is transformed into coordinates of the ultrasound coordinate system (18) and presented to the correlation unit (4).

4. Method according to at least one of the preceding claims,
**characterized in that,**
assigning the focus marking (26) is accomplished by transformation of the focus coordinates $(\tilde{x}_F, \tilde{y}_F)$ known in the ultrasound coordinate system (18) into matrix coordinates $(X_F, Y_F)$.

5. Method according to at least one of the preceding claims,
**characterized in that,**
the display image matrix (23) is segmented.

6. Method according to at least one of the preceding claims,
**characterized in that,**
the position of the ultrasound image (17) within the display image matrix (23) is determined by segmentation.

**7.** Method according to at least one of the preceding claims,
**characterized in that,**
a depth scale (21) is extracted from the display image matrix (23) by segmentation.

**8.** Method according to at least one of the preceding claims,
**characterized in that,**
the y- location of the x-axis ($X_{off}$) of the ultrasound coordinate system (18) is determined directly from the display image matrix (23).

**9.** Method according to at least one of the preceding claims,
**characterized in that,**
a scaling factor of the ultrasound image (17) is determined directly from the display image matrix (23).

**10.** Method according to at least one of the preceding claims,
**characterized in that,**
at least one arbitrary y-coordinate ($Y_1$) of the display image matrix (23) is correlated
with a y-value on the y-axis of the ultrasound coordinate system (18) by means of the depth scale (21).

**11.** Method according to at least one of the preceding claims,
**characterized in that,**
the matrix coordinates ($X_F$, $Y_F$) of the focus (F) are calculated from the focus coordinates ($\tilde{x}_F, \tilde{y}_F$) in the ultrasound coordinate system (18), the x- location of the
y-axis and the y- location of the x-axis, the orientation factors ($O_v$),($O_h$)and the
scaling factor ($S$).

**12.** Apparatus for assigning a marking indicating a focus (F) of a source of therapeutic acoustic waves (28) of a therapeutic acoustic wave device (1) to a position on an ultrasound image (17), having an ultrasound unit (2) for generating a display image (22) containing the ultrasound image (17), and a display unit (3) displaying the display image (22, 23) containing the ultrasound image (17) with a focus marking (26) assigned thereon,
**characterized in that,**
a correlation unit (4) being separate from the ultrasound unit (2) is provided for assigning a focus marking (26) to a position on the ultrasound image (17) transmitted to it on the basis of the position of the therapeutic acoustic wave source (28) relative to the ultrasound transducer (14) of the ultrasound unit (2), said correlation unit (4) being arranged between the therapeutic acoustic wave device (1), the ultrasound unit (2) and the display unit (3).

**13.** Apparatus according to claim 12,
**characterized in that,**
the correlation unit (4) further comprises a read-in unit (5) for reading in the display image (22) in a matrix format.

**14.** Apparatus according to at least one of the claims 12 and 13,
**characterized in that,**
the correlation unit (4) comprises an image recognition unit (6) for segmentation of the display image matrix (23).

**15.** Apparatus according to at least one of the claims 12 to 14,
**characterized in that,**
the correlation unit (4) further comprises a vertical scale recognition unit (101) for reading the information from the display image matrix (23).

**16.** Apparatus according to at least one of the claims 12 to 15,
**characterized in that,**
the vertical scale recognition unit (101) is capable of determining a scaling factor of the ultrasound image (17).

**17.** Apparatus according to at least one of the claims 12 to 16,
**characterized in that**
the correlation unit further comprises a horizontal orientation unit (102) for identifying the horizontal orientation of the ultrasound image (17).

**18.** Apparatus according to at least one of the claims 12 to 17,

**characterized in that,**
the correlation unit (4) further comprises a calculating unit (11) for calculating the position of the focus (F) in the display image matrix (23) on the basis of the focus coordinates $(\tilde{x}_F, \tilde{y}_F)$ in the ultrasound coordinate system (18) given to it.

19. Apparatus according to at least one of the claims 12 to 18,
**characterized in that,**
the correlation unit (4) further comprises a marking unit (12) for marking the display matrix coordinates $(X_F, Y_F)$, (26) corresponding to the focus (F).

Fig.1

```
┌──────────────┐                      ┌──────────────┐
│ Shock wave   │  relative position   │ Ultrasound   │
│ device       │ ───────────────────> │ unit         │
│ 1            │                      │ 2            │
└──────────────┘                      └──────────────┘
                                             │
                                             │ display image
                                             │ having focus F
                                             │ assigned thereon
                                             ▼
                                      ┌──────────────┐
                                      │ Display      │
                                      │ element      │
                                      │ 3            │
                                      └──────────────┘
```

Fig. 2

```
                                      ┌──────────────┐
                                      │ Ultrasound   │
                                      │ unit         │
                                      │ 2            │
                                      └──────────────┘
                                             │
                                             │ display image 22
                                             ▼
┌──────────────┐                      ┌──────────────┐
│ Shock wave   │  relative position   │ Correlation  │
│ device       │ ───────────────────> │ unit         │
│ 1            │                      │ 4            │
└──────────────┘                      └──────────────┘
                                             │
                                             │ display image
                                             │ having focus F
                                             │ assigned thereon
                                             ▼
                                      ┌──────────────┐
                                      │ Display      │
                                      │ element      │
                                      │ 3            │
                                      └──────────────┘
```

Fig. 3

22

4

| read in unit    5 |

| image recognition unit    6 |

ultrasond
grey scale
image

additional image
information
e.g. labels etc.

| center line
detection unit
100 | | vertical scale
recognition unit
101 | | horizontal
orientation unit
102 |

$X_{Off}$

$Y_{Off}, O_v, S$

$O_h$

$\widetilde{x}_F, \widetilde{y}_F$

| calculating unit    11 |

| marking unit    12 |

| Out put unit    13 |

Fig. 4a

Fig. 4b

Fig.4c

28

$\tilde{x}$

14

15

18

$\tilde{y}$

34

F

16

Fig.5

31    20    22

25

17

0    24
21
20

7 cm    24

20

20

20

Fig. 6

$X_0, Y_n$    20    23    $X_m, Y_n$

25

31

20

17

0    24
20
21

7 cm    24

$X_0, Y_0$    20    $X_m, Y_0$

Fig. 7

Fig. 8

Fig. 9

Fig. 10

$$\overline{F} = (X_{\overline{F}}, Y_{\overline{F}})$$

Fig. 11

**Europäisches Patentamt**
**European Patent Office**
**Office européen des brevets**

# EUROPEAN SEARCH REPORT

Application Number

EP 08 01 7738

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 6 221 014 B1 (BAUER EDGAR [DE]) 24 April 2001 (2001-04-24) | 1-10, 12-16, 18,19 | INV. A61B17/22 |
| Y | * column 2, line 53 - line 65 * <br> * column 3, line 20 - line 37 * <br> * column 4, line 16 - column 6, line 49 * <br> * figures 2,3,4A,4B,4C,5 * <br> ----- | 11,17 | ADD. G06T5/00 A61B19/00 |
| X | US 4 991 604 A (WURSTER HELMUT [DE] ET AL) 12 February 1991 (1991-02-12) <br><br> * column 3, line 29 - column 5, line 2 * <br> * column 6, line 48 - line 68 * <br> * figures 1,2,4 * <br> ----- | 1-5, 12-15, 18,19 | |
| Y | EP 1 643 444 A (MEDCOM GES FUER MEDIZINISCHE B [DE]; ESAOTE SPA [IT]) 5 April 2006 (2006-04-05) <br> * paragraphs [0025] - [0028], [0032] - [0034] * <br> * figures 1-4 * <br> ----- | 11,17 | |

TECHNICAL FIELDS SEARCHED (IPC)

A61B
A61N
G06T

The present search report has been drawn up for all claims

| Place of search | Date of completion of the search | Examiner |
|---|---|---|
| Munich | 8 December 2008 | Maier, Christian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

....................................................................

& : member of the same patent family, corresponding document

EPO FORM 1503 03.82 (P04C01)

**EP 2 174 600 A1**

## ANNEX TO THE EUROPEAN SEARCH REPORT
## ON EUROPEAN PATENT APPLICATION NO.

EP 08 01 7738

This annex lists the patent family members relating to the patent documents cited in the above-mentioned European search report.
The members are as contained in the European Patent Office EDP file on
The European Patent Office is in no way liable for these particulars which are merely given for the purpose of information.

08-12-2008

| Patent document cited in search report | | Publication date | Patent family member(s) | | Publication date |
|---|---|---|---|---|---|
| US 6221014 | B1 | 24-04-2001 | DE | 19648338 A1 | 04-06-1998 |
| | | | FR | 2756169 A1 | 29-05-1998 |
| | | | JP | 3614288 B2 | 26-01-2005 |
| | | | JP | 10165407 A | 23-06-1998 |
| US 4991604 | A | 12-02-1991 | DE | 3811872 A1 | 26-10-1989 |
| | | | EP | 0337056 A2 | 18-10-1989 |
| EP 1643444 | A | 05-04-2006 | AT | 404951 T | 15-08-2008 |
| | | | CN | 1760915 A | 19-04-2006 |
| | | | US | 2006072808 A1 | 06-04-2006 |

EPO FORM P0459

For more details about this annex : see Official Journal of the European Patent Office, No. 12/82

23

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- DE 3915384
- EP 1882452 A
- DE 19519956 A1
- DE 19548000